# EUROPEAN PATENT APPLICATION

(11) **EP 4 283 305 A1**
(43) Date of publication of application: **29.11.2023**
(21) Application number: 22175635.6
(22) Date of filing: 26.05.2022
(51) Int. Cl.: G01N 33/84, G01N 33/92

(54) **REGENERABLE PHOSPHOLIPID BIOSENSOR AND METHOD FOR ITS FABRICATION ON THE SURFACE OF SILANIZED OXIDES**

(71) Applicant: Vilniaus Universitetas, 01513 Vilnius (LT)
(72) Inventor: Gabri nait , Inga, LT-01513 Vilnius (LT); Vali nien , Au ra, LT-01513 Vilnius (LT); Valin ius, Gintaras, LT-01513 Vilnius (LT); ilinskas, Albinas, LT-01513 Vilnius (LT)
(74) Representative: Zaboliene, Reda

(57) **Abstract**

The membrane sensor described herein is constructed using silane compounds forming self-assembled monolayer on electrically conductive and non-conductive oxide surfaces. Self-assembled monolayer is mixed with diluents to form a surface-attached bilayer phospholipid membrane having a submembrane water reservoir. Formed sensitive phospholipid layer can be removed and formed one or more times on the same surface.

## Description

### FIELD OF THE INVENTION

The invention is related to tethered lipid bilayer membranes, in particular to the construction of a regenerable phospholipid biosensor on electrically conductive and non-conductive oxide surfaces.

### BACKGROUND OF THE INVENTION

The cell is the smallest structural unit of life. It is surrounded by a plasma membrane that protects and separates the inside of the cell from the outside. Membrane mainly consists of phospholipids - amphiphilic substances that have two hydrophobic carbon chains (tails) and a hydrophilic phosphate group (head). Phospholipids form a two-layer structure with hydrophilic heads facing outward and hydrophobic tails facing inward of the bilayer. Various types of proteins are embedded in the membrane in order to transport substances from the inside of the cell to the outside and to perform other vital functions such as respiration. Because of the membrane ability to be immobilize proteins and to mimic physical and chemical properties of live cell membranes, bilayer lipid membrane models have been developed. The composition of the membrane models can be easily controlled; therefore, different properties of the membrane can be achieved.

Membrane models are usually applied as a platform to study protein-membrane interactions, including integral proteins (pore-forming toxins, ion channels) and peripheral proteins (enzymes, electron transporters). Electrochemical methods, together with microscopic and spectral methods, are very effective to study membrane processes, but in order to apply such methods, membrane models should be formed on solid electrically conductive surfaces (Ragaliauskas T. et al., 2017).

One of the most convenient methods of forming a membrane on a solid surface is by vesicles fusion. Vesicles are liposomes with a spherical structure composed of a bilayer of phospholipids. When the vesicle solution is poured directly onto the test surface, a self-assembly of the lipids occurs, with the phospholipids transitioning from the spherical form onto flat surface to form a bilayer-planar structure. Because the membrane forming method is based on self-assembly, it does not require any expensive equipment, making it a cheap and affordable method. This method of membrane formation is also convenient because the composition of the bilayer can be easily controlled by changing the lipid composition in the vesicles.

One of the simplest membrane models is the solid supported lipid membrane. It is composed of two layers of phospholipids, but, unlike a cell that is spherically shaped, this bilayer is immobilized in a horizontal plane (Castellana E.T. et al., 2006). The flat lipid membrane is characterized by a very thin layer of water between the membrane and the hard surface. This layer of water "mimics" the inside of the cell. With such model system, proteins can be immobilized and various studies performed to elucidate membrane permeability, heterogeneity and other properties. Although this membrane model has moderate stability, various defects in the membrane and their abundance do not allow to apply electrochemical measurement methods to assess the integrity of flat membranes and to use such membranes for electrochemical biosensors to measure the activity of bacterial toxins and other membrane-damaging agents. Hybrid membranes (Plant A.L. 1999) have incomparably fewer defects. In them, the layer proximal to the surface consists of a self-assembled monolayer (SAM) and the second layer consists of phospholipids. SAM consists of molecules containing a chain of carbon atoms with a so-called active functional group (head) on one side, which is usually a thiol or trialkoxysilane functional group. SAM adheres to the surface of the functional group with the surface by forming strong covalent bonds. The selection of the active SAM functional group depends on the nature of the substrate that would be used for SAM formation. If a gold surface is used, the thiol group containing the SAM shall be selected, and if the metal oxide surface is used, the SAM with the trialkoxysilane group shall be used. One of the main advantages of hybrid membranes is that they have a very high stability (compared to flat lipid membranes) and a small number of defects. As a result, various research methods, including electrochemical methods, can be used in dynamic experiments. However, the hybrid membrane also has a disadvantage: it does not contain a thin layer of water between the membrane and the solid surface, which would allow the incorporation of integral proteins and the use of such bilayers for biosensors measuring the activity of bacterial toxins that penetrate the membranes. Therefore, another membrane model has been developed: tethered bilayer lipid membranes (tBLMs) (Junghans A. et al., 2010; McGillivray D.J. et al., 2007). tBLMs consist of anchor SAM, resembling the lipid structure. These anchors also have an active functional group that forms strong bonds with the substrate, as in conventional SAM, but the tail below consists of a polyethylene glycol (PEG) chain and two chains of carbon atoms that resemble the hydrophobic part of the lipid molecule. During vesicles fusion process, a thin layer of water is formed between solid surface and SAM due to existence of PEGs in anchor SAM. To incorporate toxins or other proteins into membrane, the amount of anchors on the surface must be diluted using short-chain low molecular weight SAM compounds that have a hydrophilic group on the outside (such as a hydroxy group). The formation of a mixed anchor SAM from long and short-chain compounds along with vesicle fusion results in a tethered bilayer lipid membrane with ionic water reservoir. This system can be applied for integral protein research, detection of pore-forming toxins.

Membranes can be formed on different surfaces. One of the most used substrates is the atomically smooth gold, which is usually prepared by magnetron sputtering. Atomically smooth surfaces can be investigated using atomic force microscopy (AFM), surface plasmon resonance (SPR) or neutron reflectometry (NR) methods. Prepared membranes exhibit low number of defects and high stability. The thickness of the ionic water reservoir is 2 nm, which was determined by neutron reflectometry (McGillivray D.J. et al., 2007). Therefore, these membranes were successfully applied to the study pore-forming toxins and to the development of biosensors for the detection of bacterial toxins concentration.

Membrane models on sputtered gold surface are well investigated system. The optimal conditions for the membrane formation were determined, providing the first steps towards the commercialization of membrane-based biosensors (patent no. LT6427B (2017) "Method for obtaining tethered bilayer lipid membranes (tBLM)"). Knowing that gold and the surface preparation process (magnetron sputtering) are expensive, the goal is to discover alternative substrates that would be cheaper and commercially available to create a cheap and easy-to-prepare biosensor. Also, membrane models on metallic substrates such as gold are opaque, making it difficult to apply optical methods for technologic development involving applications of tBLMs. It is important to note that there is evidence (Rakovska et al., 2015) that anchor SAM - gold thiolates in contact with water environment detach from and migrate on the gold surface making clusters of molecular anchors which leads to deterioration of tBLMs, thus preventing regeneration of tBLMs in aqueous media.

An alternative to gold substrates could be thin film metal oxides. Recently, tin oxide doped with different elements has received more attention from scientific community. Thin film tin oxide is optically transparent and electrically conductive semiconductor, which means that lipid membranes formed on tin oxides can be investigated with the optical techniques in a transmission mode as well as can be used in photovoltaics. Also, one may expect from the significantly higher melting temperature of many conducting metal oxides, the propensity of detachment of surface atoms from metal oxide surface will be much less compared to surfaces of gold.

In order to form tethered bilayer lipid membrane on metal oxide surfaces, a self-assembling monolayer consisting of trialkoxysilanes must be used. Strong Si-O-metal covalent bonds are formed during SAM formation: trialkoxysilanes are hydrolyzed during spontaneous non-reversible reaction (Wasserman S.R. et al., 1989). In order to form a membrane on metal oxide surfaces that has a submembrane water reservoir and can be applied for pore-forming toxins and other integral protein studies, it is necessary to find molecular anchors for SAM formation that have a hydrophilic moiety attached to functional group (that attaches to surface of interest) and is continued by hydrophobic tail. Formation of SAM with hydrophilic moiety, would form a thin layer of water in the hydrophilic part due to hydrogen bonds, and the hydrophobic part would anchor the lipids during the formation of phospholipid membrane.

Thus, in summary, the main disadvantages of the application of surface-immobilized membranes in the development of biosensors can be identified as:
1. Gold substrates that are being used for the membrane formation are opaque, making it more difficult to apply optical methods for investigation. Meanwhile, oxide substrates can be made optically transparent.
2. Lipid membranes can be formed on gold surfaces only once, without deteriorating properties of the membrane i.e., due to the lability of gold thiolates they tend to migrate on the surface, thus membrane regeneration becomes impossible. Therefore, it is relevant to apply oxidic surfaces for membrane formation, that can be regenerated multiple times.
3. tBLMs formed on gold surface functionalized with thiol SAMs can be utilized in relatively narrow interval of potentials (optimal interval: from -0.1 V to 0.5 V *vs* Ag/AgCl,Cl⁻_{sat.}) without irreversible deterioration of the electrical properties of tBLMs, which limits the utility of biosensing devices.
4. Using thiolate chemistry to form anchor SAMs, the range of substrates is limited to metals such as gold, while silane anchor compounds allow the list of substrates to be extended to semiconductors and dielectrics.
5. The commonly used surface - gold, is an expensive metal, expensive equipment is used for its preparation, therefore the aim is to apply cheaper electrically conductive and non-conductive oxide along with oxidized metal surfaces.

### BRIEF DESCRIPTION OF THE INVENTION

### Abbreviations:

EIS - electrochemical impedance spectroscopy
CV - cyclic voltammetry
AFM - atomic force microscopy
SPR - surface plasmon resonance
NR - neutron reflectometry
SAM - self-assembled monolayer
PEG - polyethylene glycol
NMR - nuclear magnetic resonance
tBLM - tethered bilayer lipid membrane

Surfaces of metals and non-metal oxides have been selected for the implementation of present invention. The application of thin film oxide surfaces for membrane formation has advantages over the gold film surfaces that are usually used for the formation of membranes. The latter are expensive, sometime require have a minimum thickness of 50 nm and more and as such are not optically transparent. Because of the optical transparency of thin-film oxides, the formed phospholipid membranes can be studied by optical techniques and potentially be applied to photocurrent generation or for the development of solar cells.

Due to the chemistry of the oxide surface, silane compounds are used to form a self-assembling monolayer by forming strong Si-O-metal bonds with the surface of the metal oxides, which ensures that the formed self-assembled monolayer is highly stable. For this reason, silanized oxide surfaces should be suitable for multiple formation of phospholipid membranes with the same properties, as opposed to gold film surfaces with surface mobility of gold thiolates, resulting in destabilization of anchor monolayers and formation of clusters. This leads to degradation of membrane functional integrity.

In this work, a new silane (with either one or two polymethylene chains) synthesis procedure based on thiol-ene click-reaction chemistry that does not require expensive equipment was used to form SAM. Either single chain thiols or thiolipids (with hydrophilic ethylene oxide chain and hydrophobic two polymethylene chains [McGillivray D. et al, 2007, Budvytyte R. et al. 2013, Junghans A. et al. 2010]) could be used to synthesize new anchor silanes. Either the mixture of long-chain and short-chain silanes or pure silanes prepared during the synthesis can be immediately used to form a monolayer without further purification. By changing the composition of SAM the sensitivity of biosensor can be manipulated. Phospholipid membranes formed during vesicle fusion are stable and can be regenerated.

Metal or non-metal oxides are less expensive than Au surfaces usually used to form phospholipid membranes, therefore, commercial application of phospholipid biosensors designed to detect toxins and other biologically active substances on electrically conductive and non-conductive oxide surfaces, becomes more accessible.

Advantages of the proposed method:
1. The application of silane anchors (with either one or two polymethylene chains) can be used to construct phospholipid biosensors on inexpensive, as well as commercially available, electrically conductive and non-conductive surfaces of oxidic and oxidized metals.
2. Single chain molecular anchor can be replaced with lipid molecular anchor to increase the sensitivity of the biosensor to pore forming toxins.
3. The proposed biosensor may be regenerated and reused several or more times.
4. The proposed biosensor is stable in a wide range of potentials (dependent on substrate material, in case of FTO from 0 V to 1 V vs Ag/AgCl,Cl⁻_{sat.}) that expands the applicability in biosensing devices.
5. The proposed biosensor can be constructed on an optically transparent substrate and integrated into optical devices/methods.
6. The proposed biosensor can be constructed on conductive, semiconductor and dielectric surfaces.

### BRIEF DESCRIPTION OF THE DRAWINGS

**Figure 1****.** Electrochemical impedance spectra in Cole - Cole plot at 0 V potential vs Ag/AgCl,Cl⁻_{sat.}: filled circles - pristine FTO glass, open circles - FTO glass functionalized with TOPS:ATS 1:1 SAM in heptane, filled triangles - tBLM , composition - DOPC/Chol 6:4.
**Figure 2****.** Electrochemical impedance spectra in Cole - Cole plot at 0 V potential vs Ag/AgCl,Cl⁻_{sat.}: filled circles - pristine FTO glass, open circles - FTO glass functionalized with TOPS:ATS 1:1 SAM in toluene, filled triangles - tBLM , composition - DOPC/Chol 6:4.
**Figure 3****.** Electrochemical impedance spectra in Cole - Cole plot at 0 V potential vs Ag/AgCl,Cl⁻_{sat.}: filled circles - pristine ITO glass, open circles - ITO glass functionalized with TOPS:ATS 1:1 SAM in heptane, filled triangles - tBLM , composition - DOPC/Chol 6:4.
**Figure 4****.** Fluorescence microscope images, A - DOPC/Chol 6:4 tBLM, (where 1% cholesterol is labeled with fluorescent dye (Chol-Cy5)) on TOPS:ATS 1:1 SAM functionalized microscope slides, B - same membrane on the microscope slide plates that were not functionalized by SAM.
**Figure 5****.** Electrochemical impedance spectra in Cole - Cole plot at 0 V potential vs Ag/AgCl,Cl⁻_{sat.}: filled circles - pure SS plate, open circles - SS plate functionalized with TOPS:ATS 1:1 SAM in heptane solvent, filled triangles - tBLM, composition - DOPC/Chol 6:4. Inset A is the zoomed-in part of the curve.
**Figure 6****.** Electrochemical impedance spectra in Cole - Cole plot at 0 V potential vs Ag/AgCl,Cl⁻_{sat.}: filled circles - sputtered Ti/TiO₂ plate, open circles - sputtered Ti/TiO₂ plate functionalized with TOPS:ATS 1:1 SAM in heptane solvent, filled triangles - tBLM, composition - DOPC/Chol 6:4. A is the zoomed-in part of the curve.
**Figure 7****.** Electrochemical impedance spectra in Cole - Cole plot at 0 V potential vs Ag/AgCl,Cl⁻_{sat.}: filled circles - Si plate, open circles - Si plate functionalized with TOPS:ATS 1:1 SAM in heptane, filled triangles - tBLM , composition - DOPC / Chol 6: 4.
**Figure 8****.** Electrochemical impedance spectra in Cole - Cole plot at 0 V potential vs Ag/AgCl,Cl⁻_{sat.}: filled circles - DOPC/Chol 6:4 tBLM formed on FTO functionalized TOPS:ATS 1:1 SAM, open circles - after 60 min of 50 nM melitin insertion into the membrane, filled triangles - after 60 min of 100 nM melittin insertion into the membrane. A is the zoomed-in part of the curve
**Figure 9****.** Electrochemical impedance spectra in Cole - Cole plot at 0 V potential vs Ag/AgCl,Cl⁻_{sat.}: filled circles - DOPC tBLM formed on FTO functionalized TOPS:ATS 1:1 SAM, open circles - after 60 min of 50 nM melitin insertion into the membrane, filled triangles - after 60 min of 100 nM melittin insertion into the membrane.
**Figure 10****.** Dependence of the change in tBLM conductivity on the incorporation of different melittin concentrations into the DOPC:Chol 6:4 membrane.
**Figure 11****.** Electrochemical impedance spectra in Cole - Cole plot at 0 V potential vs Ag/AgCl,Cl⁻_{sat.}: filled circles - DOPC/Chol 6:4 tBLM formed on FTO functionalized TOPS: ATS 1:1 SAM, open circles - after 60 min of 50 nM α-hemolysin insertion into the membrane, filled triangles - after 60 min of 100 nM α-hemolysin insertion into the membrane. A is the zoomed-in part of the curve.
**Figure 12****.** Electrochemical impedance spectra in Cole - Cole plot at 0 V potential vs Ag/AgCl,Cl⁻_{sat.}: filled circles - DOPC tBLM formed on FTO functionalized with TOPS:ATS 1:1 SAM, white circles - after 60 min of 100 nM α-hemolysin incorporation into the membrane.
**Figure 13****.** Dependence of the change in tBLM(DOPC/Chol 6:4), formed on TOPS:ATS SAM, conductivity on the incorporation of different α-hemolysin concentrations into the DOPC/Chol 6:4 membrane.
**Figure 14**. Electrochemical impedance spectra in Cole - Cole plot at 0 V potential vs Ag/AgCl,Cl⁻_{sat.}: open symbols - formation of the membrane several times; filled symbols - removal of the membrane.
**Figure 15****.** Electrochemical impedance spectra in Cole - Cole plot at 0 V potential vs Ag/AgCl,Cl⁻_{sat.}: filled circles - clear FTO glass, open circles - FTO glass functionalized with WC14-S and ATS SAM in toluene, filled triangles - tBLM , composition - DOPC/Chol 6:4.
**Figure 16****.** Electrochemical impedance spectra in Cole - Cole plot at 0 V potential vs Ag/AgCl,Cl⁻_{sat.}: filled circles - DOPC/Chol 6:4 tBLM formed on FTO functionalized WC14-S and ATS SAM, open circles - after 30 min of 100 nM α-hemolysin insertion into the membrane, filled triangles - after 30 min of 200 nM α-hemolysin insertion into the membrane.
**Figure 17****.** Dependence of the change of tBLM (DOPC/Chol 6:4), formed on WC14-S and ATS SAM (filled circles) and tBLM (DOPC/Chol 6:4), formed on TOPS: ATS SAM (filled triangles) conductivity on the incorporation of different α-hemolysin concentrations into the DOPC/Chol 6:4 membrane.
**Figure 18****.** Electrochemical impedance spectra in Cole-Cole plot of tBLM (DOPC/Chol 6:4), formed on WC14-S and ATS SAM, at different potentials vs Ag/AgCl,Cl⁻_{sat.} (from 0 V to 1 V vs Ag/AgCl,Cl⁻ₛₐₜ).
**Figure 19****.** Electrochemical impedance spectra in Cole - Cole plot at 0 V potential vs Ag/AgCl,Cl⁻_{sat.}: filled circles - DOPC/Chol 6:4 tBLM formed on FTO functionalized with TOPS:APTES 1:1 SAM, after 30 min insertion of different melittin concentration: open circles - 50 nM, filled squares - 100 nM , open squares - 200 nM, filled triangles - 300 nM.
**Figure 20****.** Dependence of the change in tBLM (DOPC/Chol 6:4), formed on TOPS:APTES 1:1 SAM, conductivity on the incorporation of different melittin concentrations into the DOPC/Chol 6:4 membrane.

### DETAILED DESCRIPTION OF THE INVENTION

Due to the fact that the previous phospholipid biosensors used a gold surface that is not optically transparent, furthermore, the formed self-assembled alkylthiol monolayers and phospholipid membranes on the gold surface are not sufficiently stable and cannot be regenerated. Also, considering the cost of gold, the surfaces of metals and non-metal oxides were selected for the implementation of the present invention. The first advantage of using oxides is that they can be produced as thin films that have a high transmittance of visible light and UV radiation.

This feature allows the application of optical methods in the development of biosensors, which could not be done with gold surfaces. Optical methods expand the potential scope of research and pave the way for the application of phospholipid membranes on solid surfaces in the development of photocurrent generating systems or light-harvesting systems.

In order to form a phospholipid biosensor, one requires a hydrophobic self-assembled monolayer that has the ability to adsorb and fuse phospholipid vesicles to form a uniform, nearly defect-free phospholipid bilayer. Au surface is usually functionalized with alkylthiol SAM, where covalent Au-S bonds are formed. Although Au-S bonds are particularly strong, due to the lability of the surface gold atoms, the monolayer molecules are mobile on the surface, as well. During prolonged contact with the aqueous medium (up to several hours), monolayer forms clusters, that is no longer uniform. This interferes with the formation of uniform phospholipid bilayer. Meanwhile, SAMs, that were formed from trichlorosilane (or trialkoxysilane) on metal oxide surfaces, are highly stable due to the surface chemistry of the oxides - strong Si-O-metal covalent bonds are formed (Wasserman S.R. et al., 1989). Prolonged contact with the aqueous medium or with other physical external factors has little or no effect on the properties of the monolayer. This provides additional advantages in the use of oxidic surfaces over the use of Au surfaces for the formation of phospholipid membranes: the membranes not only have a high stability over a long period of time (several days), but also the possibility to regenerate the membranes on the same surface without deteriorating the membrane properties. Also, given the cost of preparing gold surfaces, oxide surfaces of metals and non-metals are significantly cheaper, which is relevant for the commercial application of a phospholipid biosensor for the detection of toxins and other membrane proteins and biologically active substances.

The lack of commercially available trichlorosilane anchors has been taken into account in the present invention for the application of metal and non-metal oxides to the formation of phospholipid biosensors. Also, the ability to manipulate the sensitivity of the biosensor has been taken into account, as well. Based on click-reaction chemistry that does not require expensive equipment, the synthesis of new silanes was performed. This technique is superior to the already known synthesis methods in that a mixture of long-chain and short-chain silane or pure silane is prepared, where the short-chain dilutes the amount of long anchors on the surface during self-assembly monolayer to form a sparsely populated self-assembled monolayer. This allows to form a tethered bilayer lipid membrane.

### Materials and methods

Fluorine doped tin oxide - FTO, 300 mm x 300 mm x 2.2 mm (Sigma-Aldrich, Germany) Indium tin oxide - ITO, 150 mm x 150 mm x 0.7 mm (Delta technologies, limited, USA) Stainless steel - SS, 316L alloy composition: Fe/Cr 18%, Ni 10%, Mo 3%, 150 mm x 150 mm x 0,5 mm (Goodfellow Cambridge Limited, United Kingdom)
Glass slide - 25 mm x 75 mm (Thermo Fisher)
Trichloro(3-(octadecylthio)propil)silane - TOPS
20-tetradecyloxy-3,6,9,12,15,18,22-heptaoxahexatricontane-1-thiol - WC14 (McGillivray D. et al, 2007)
1,1,1-trichloro-25-(tetradecyloxy)-8,11,14,17,20,23,27-heptaoxa-5-thia-1-silahentetracontane - WC14-S
Deionised water - H₂O (Adrona Sia, Latvia)
Sodium chloride - NaCl (FLUKA, Switzerland);
Sodium dihydrophosphate - NaH₂PO₄·2H₂O (FLUKA, Switzerland)
Sodium hydroxide - NaOH (Chempur, Germany)
Allyltrichlorosilane - H₂C=CHCH₂SiCl₃, ATS (Aldrich, Germany)
Aminopropyltriethoxysilane - APTES (Aldrich, Germany)
Octadecanthiol - CH₃(CH₂)₁₇SH, ODT (Aldrich, Germany)
2,2-dimethoxi-2-phenylacetophenone - C₁₆H₁₆O₃, DMPA (Acros Organics, USA)
Heptane - C₇H₁₆ (Roth, Germany)
Toluene -C₇H₈ (Roth, Germany)
Chloroform - CHCl₃ (Sigma-Aldrich, Germany)
1,2-dioleoyl-3-phosphoglicerocholyne - DOPC (Avanti Polar Lipids, USA)
Cholesterol - chol (Avanti Polar Lipids, USA)
Melittin - mel, from honey bee venom (Sigma-Aldrich, Germany)
α-hemolysin - aHL, from *Staphylococcus aureus* (Sigma-Aldrich, Germany)
Cholesterol with merocyanine dye: 2-(1E,3E,5E)-5-(1-(5-carboxipentil)-3,3-dimetilindolin-2-ilidene)penta-1,3,3-trimetil-3H-indole bromide - Chol-Cy5 (Eicher-Lorka O. et al., 2016)
WC14 - 20-tetradecyloxy-3,6,9,12,15,18,22-heptaoxahexatricontane-1-thiol
UV lamp - 365 nM 5W

¹³C and ¹H NMR spectra were recorded with a Bruker Ascend 400 spectrometer (400 MHz frequency ¹H NMR and 100 MHz - ¹³C NMR) using residual solvent (CDCl₃) signals. Chemical shift is given on the δ scale (ppm).

EIS measurements were performed with a potentiostat/galvanostat utAutolab Type III (Metrohm Lab, The Netherlands) with FRA software installed. Measurements were performed in a standard three-electrode cell, in which the working electrode was a metal oxide surface (working area 0.32 cm²), the reference was Ag/AgCl,Cl⁻_{sat.} (Microelectrode Model M-401 F, Bedford, NH), auxiliary platinum wire (99.99%, Aldrich, Germany). The phosphate buffer used for the electrochemical measurements consisted of 0.01 M NaH₂PO₄ and 0.1 M NaCl, adjusting the pH to 7.1 with NaOH.

Fluorescence microscopy was performed using an Olympus BX61WI fluorescence microscope, a water immersion objective UMPlanFN-W (10X / 0.30 NA) and a Qimaging EXi Aqua camera. An Hg lamp and an Olympus U-MWG2 filter were used to determine the surface distribution of Chol-Cy5. The glass slides were mounted on the bottom of Petri dishes and the vesicle solution was completely eluted from the system with clean PBS pH 7.1 before recording the photos.

### Description of the process

### Example 1

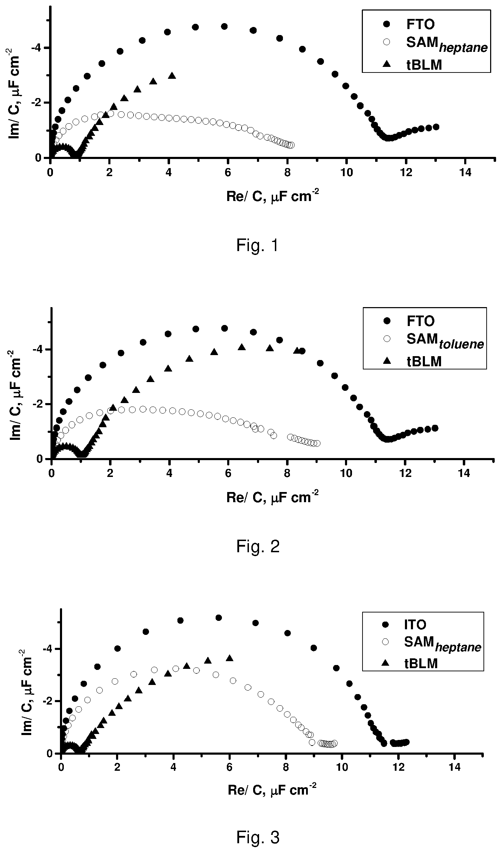

1. The synthesis of the TOPS is performed by a click-reaction mechanism (Tucker-Schwartz A. K. et al., 2011). ATS and ODT are mixed in a 2:1 molar ratio in a clear tube. Then, 2 mol% DMPA (photoinitiator) are added and stirred on a magnetic stirrer until a homogeneous mixture is obtained. This mixture is irradiated with a 5 W UV lamp at 365 nm for 24 hours. After this procedure, we have a ready-to-use silanization mixture (TOPS:ATS 1:1) for SAM formation. The TOPS:ATS 1:1 mixture is kept wrapped in aluminum foil to avoid direct sunlight, which may reduce the chemical activity of the synthesized mixture.
2. Prior to membrane formation, the FTO-coated glass plate was cut into 25 mm x 10 mm electrodes and thoroughly cleaned: incubation in an ultrasonic bath in 2 different media for 10 minutes: (i) in 2% Micro 90 solution; (ii) in deionized water. Next, the FTO plate is incubated conc. H₂SO₄ for 1 h, then rinsed with deionized H₂O and incubated in 2-propanol in an ultrasonic bath for 10 min. In the last step, the FTO is incubated in deionized H₂O at about 18-20 hours. (overnight) and dried in a stream of N₂ gas.
3. The prepared sample is then used in the SAM formation - silanization procedure. 20 ml of heptane (or other saturated alkane) is heated in a small beaker to 60 °C and added 30 µl of the synthesized TOPS: ATS mixture. The electrode is then placed in a vertical position in the stirred silanization solution for 1 hour, at 60 ° C. The sample is then washed with pure heptane, dried under a stream of N₂ gas and heated at 100 °C for 1 h.
4. The formation of tBLM is completed using a vesicle fusion method (Fig. 1) (Patent No. LT6427B (2017)). 10 mM solutions of lipids in chloroform are prepared and stored at -20 °C. Then, a certain amount of lipid solution is transferred to another vial to prepare the vesicle solution of desired volume at a concentration of 1 mM. The chloroform is evaporated with a stream of nitrogen gas (about 30 minutes) until a white lipid film remained on the bottom of the vial. Then, lipid film is rehydrated with a buffer solution at pH of 4.4 and a composition of 0.01 M NaH₂PO₄ and 0.1 M NaCl. Using an automatic pipette, cycles of suction and release are repeated, until the lipid film is fully rehydrated and mixed in the solution, which has an opaque, milky white appearance.
5. Electrochemical impedance measurements were registered at bias electrode potential of 0 V vs Ag,AgCl/Cl⁻_{sat.}.

### Example 2

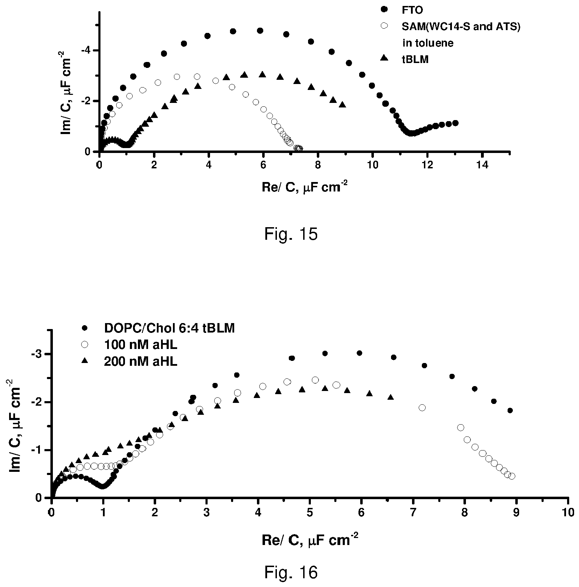

1. The procedure described in Example 1 is carried out, except in section 2 instead of ODT, WC14 is used and WC14:ATS are mixed at equimolar ratio, synthesis product - WC14-S is obtained
2. Instead of heptane, 10 ml of toluene is heated to 30 °C in section 3. Then, 9 µl of WC14-S and 2 µl of ATS is added to toluene and the electrode is placed in horizontal position, instead of vertical and kept in silanization solution for 1 h without stirring. The formation of tBLM was carried out as explained in example 1, section 4 (Fig. 15).

### Example 3

1. The procedure described in Example 1 is carried out, except that the silanization in section 3 is carried out in an aromatic solvent (e.g. toluene or benzene) (Fig. 2).

### Example 4

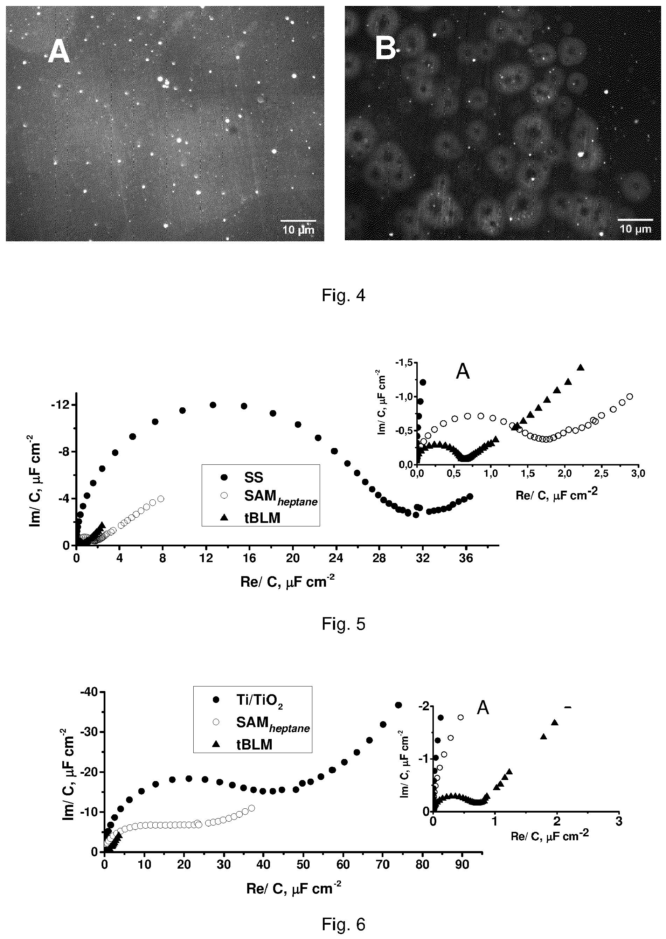

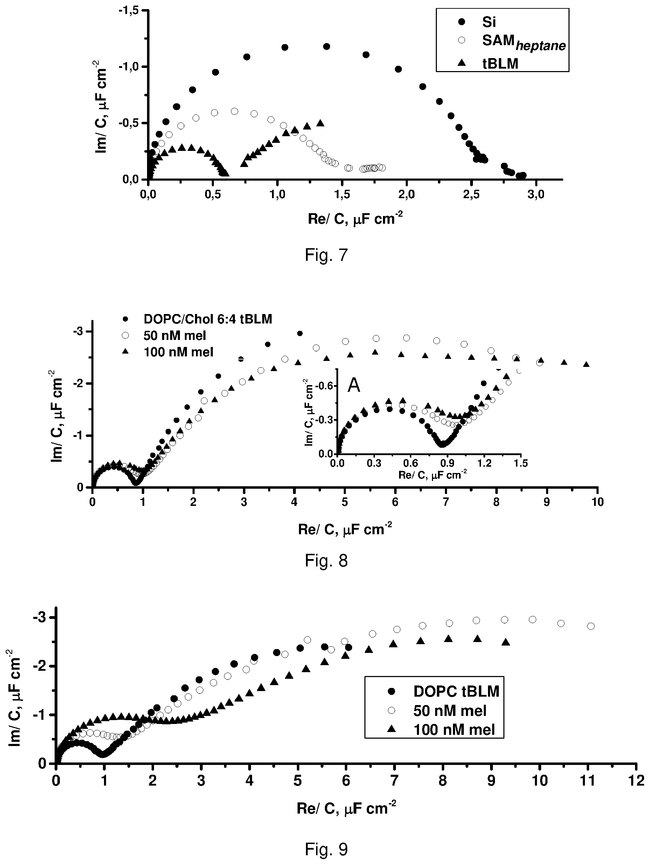

1. The procedure is as described in Example 1, except that the following is used in section 2:
(i) ITO glass (Fig. 3), but other oxide-coated glass panes may be used: cadmium tin oxide (Cd₂SnO₄), zinc oxide (ZnO), aluminum doped zinc oxide (AI:ZnO), antimony tin oxide (SnO₂/Sb₂O₅), indium antimony oxide (InSbO), gallium zinc oxide (GaZnO), indium zinc oxide (InZnO), antimony indium tin oxide (InSbSnO), indium gallium oxide (InGaZnO), bismuth selenite (Bi₂SeO₅), metal oxide perovskites;
(ii) glass plate (Fig. 4), colored glass, quartz, sapphire, mica may also be used;
(iii) stainless steel (Fig. 5), aluminum, titanium, copper, nickel, nickel-titanium alloys, cobalt and cobalt alloys may also be used;
(iv) evaporated Ti/TiO₂ glass slide (Fig. 6), evaporated Zn/ZnO, Zr/ZrO₂, Cr/CrₓO_{y} glass slide can also be used;
(v) Si/SiO₂ plate (Fig. 7), Ge/GeO₂ can also be used.

### Example 5

1. Synthesis procedure described in Example 1, section 1 is carried out, except ODT and ATS are mixed at equimolar ratio. After this procedure we have pure TOPS.
2. Silanization procedure described in example 1, section 3 is carried out, except TOPS is mixed with APTES at molar ratio 1:1.

### Example 6

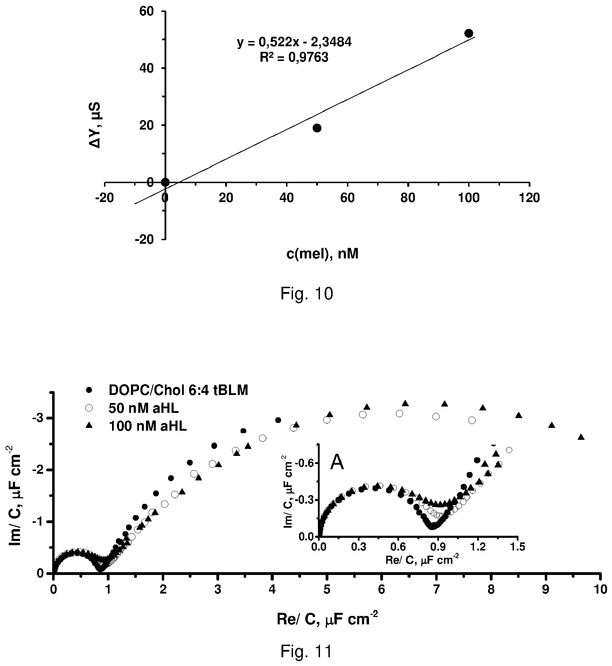

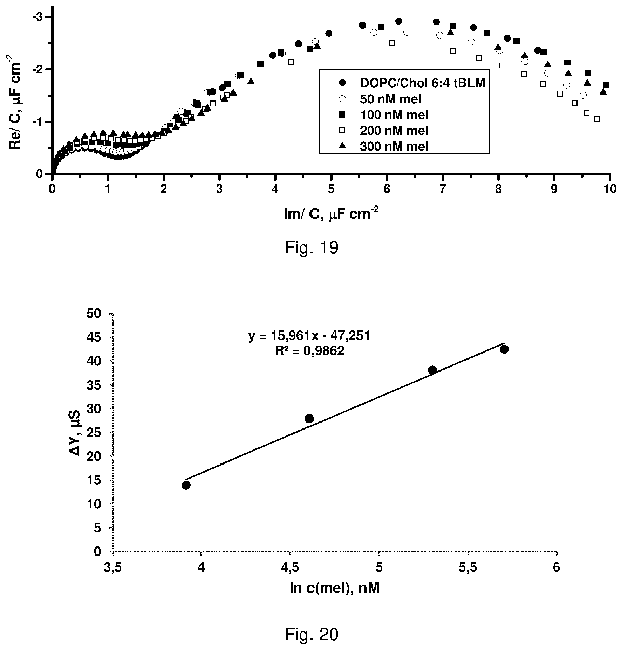

1. The procedure is as described in Example 1, only DOPC/Chol 6:4 and DOPC membrane damage with different concentrations of pore-forming toxins is performed: (i) melittin (Fig. 8, 9 and 19); (ii) α-hemolysin (Fig. 11, 12 and 16); calibration curve (toxin concentration *vs* the change in membrane conductivity) is plotted (Fig. 10, 13, 17 and 20).

### Example 7

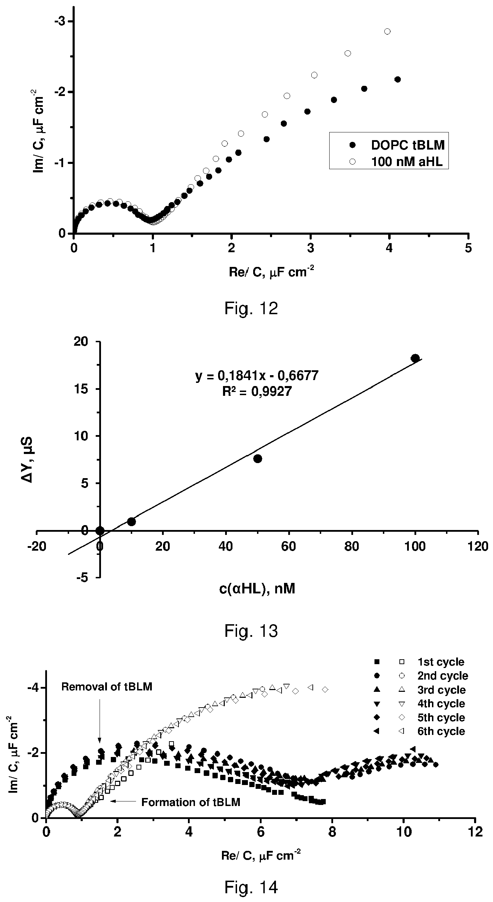

1. As described in Example 1, only the removal and formation of the phospholipid bilayer on the same surface is observed several times (Fig. 14).

### Example 8

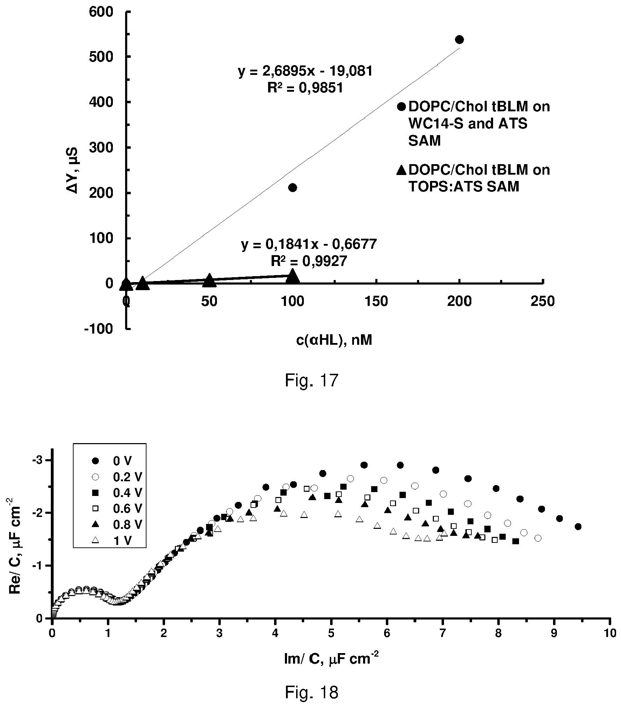

1. As described in example 1, section 5, electrochemical impedance measurements of DOPC/Chol 6:4 tBLM is recorded at different potentials, from 0 V to 1 V vs Ag,AgCl/Cl⁻_{sat.}, without deteriorating the properties of the tBLM (Fig. 18).

### Summary

The following examples show that a click-reaction chemical process can be used to synthesize a usable silane mixture or pure silanes in which a metal oxide surface, such as a glass plate coated with an FTO layer, or an ITO layer, or pure glass, colored glass, quartz, sapphire plate, or stainless steel, aluminum, titanium, copper, nickel, nickel-titanium alloy, cobalt and cobalt alloy, or evaporated Ti/TiO₂ plate and Zn/ZnO, Zr/ZrO₂, Cr/CrₓO_{y}, or Si/SiO₂ plate, or other metal or semiconductor product coated with an oxide, such as cadmium tin oxide (Cd₂SnO₄), zinc oxide (ZnO), aluminum alloyed zinc oxide (AI:ZnO), antimony tin oxide (SnO₂/Sb₂O₅), indium antimony oxide (InSbO), gallium zinc oxide (GaZnO), indium zinc oxide (InZnO) antimony indium tin oxide (InSbSnO), indium gallium zinc oxide (InGaZnO), bismuth selenite (Bi₂SeO₅), yttrium barium cuprate, perovskite, resulting in an anchor self-assembled monolayer suitable for the formation of tethered bilayer lipid membrane, tBLM the method of vesicles fusion is described in U.S. Pat. LT6427B (2017). The composition of the tBLM can be easily varied by adjusting proper lipid composition of vesicles, and the resulting tBLM can be easily regenerated without losing the functional properties of the tBLM. The phospholipid biosensors are suitable for the determination of pore-forming toxin concentration using a calibration curve, such as the dependence of the change in membrane conductivity on the toxin concentration. The sensitivity of the biosensor can be manipulated by changing conductivity of thin water layer between solid surface and the tBLM. By using lipid SAM the sensitivity of biosensor towards pore forming toxins increased 20-fold (Fig. 17).

### References

1. R. Budvytyte, G. Valincius, G. Niaura, V. Voiciuk, M. Mickevicius, H. Chapman, H. Goh, P. Shekhar, F. Heinrich, S. Shenoy, M. Lösche, D.J. Vanderah, Structure and Properties of Tethered Bilayer Lipid Membranes with Unsaturated Anchor Molecules, Langmuir 29 (2013) 8645-8656.
2. Castellana E. T, Cremer P. S., Solid supported lipid bilayers: From biophysical studies to sensor design, Surface Science Reports 61 (2006) 429-444.
3. Eicher-Lorka O., Charkova T., Matijoǎka A., Kuodis Z., Urbelis G., Penkauskas T., Mickevicius M., Bulovas A., Valincius G., Cholesterol-based tethers and markers for model membranes investigation, Chemistry and Physics of Lipids 195 (2016) 71-86.
4. Junghans A., Köper I., Structural Analysis of Tethered Bilayer Lipid Membranes, Langmuir 26 (2010) 11035-11040.
5. McGillivray D. J., Valincius G., Vanderah D. J., Febo-Ayala W., Woodward J. T, Heinrich F., Kasianowicz J. J., Lösche M., Molecular-scale structural and functional characterization of sparsely tethered bilayer lipid membranes, Biointerphases 2 (2007) 21-33.
6. Plant A.L., Supported Hybrid Bilayer Membranes as Rugged Cell Membrane Mimics, Langmuir 15 (1999) 5128-5135.
7. Ragaliauskas T., Mickevicius M., Rakovska B., Penkauskas T., Vanderah D. J., Heinrich F., Valincius G., Fast formation of low-defect-density tethered bilayers by fusion of multilamellar vesicles. Biochimica et Biophysica Acta 1859 (2017) 669-678.
8. Rakovska B. Ragaliauskas, T., Mickevicius, M., Jankunec, M., Niaura, G., Vanderah, D.J., Valincius G.. Structure and Function of Membrane Anchoring Self-Assembled Monolayers. Langmuir, 31, (2015) 846-857
9. Tucker-Schwartz A. K., Farrell R. A., Garrell R. L., Thiol-ene Click Reaction as a General Route to Functional Trialkoxysilanes for Surface Coating Applications, Journal of American Chemical Society 133 (2011) 11026-11029.
10.Wasserman S. R., Tao Y. T., Whitesides G. M., Structure and Reactivity of Alkylsiloxane Monolayers Formed by Reaction of Alkyltrichlorosilanes on Silicon Substrates, Langmuir, 5 (1989) 1074-1087.

## Claims

1. A membrane biosensor comprising silane compounds that form an anchor self-assembled monolayer of silane-organic molecules that forms a uniform phospholipid bilayer.

2. The membrane biosensor according to claim 1, **characterized in that** anchor self-assembled monolayer of silane-organic molecules are replaced by lipid-like molecules that increase the conductivity of thin water reservoir between solid support and the membrane.

3. The membrane biosensor according to claim 1, **characterized in that** organic solvents such as alkanes and / or mixtures thereof including heptane, aromatic hydrocarbons and / or mixtures thereof including toluene and benzene are used to form the self-organizing monolayer.

4. Biosensor according to claims 1-3, **characterized in that** the conductive and optically transparent metal oxides of thin films used for film formation are selected from FTO, ITO, cadmium tin oxide (Cd₂SnO₄), zinc oxide (ZnO), aluminum alloyed zinc oxide (AI:ZnO), antimony tin oxide (SnO₂/Sb₂O₅), indium antimony oxide (InSbO), gallium zinc oxide (GaZnO), indium zinc oxide (InZnO), antimony indium tin oxide (InSbSnO), indium gallium oxide (InGaZnO), bism (Bi₂SeO₅), yttrium barium cuprates, metal oxide perovskites.

5. Biosensor according to claims 1-3, **characterized in that** oxide dielectrics selected from glass, colored glass, quartz, sapphire, mica are used for membrane formation.

6. The biosensor according to claims 1-3, **characterized in that** the metallurgical surfaces of the oxidized metals used for forming the membrane are selected from stainless steel, aluminum, titanium, copper, nickel, nickel-titanium alloys, cobalt and cobalt alloys.

7. The biosensor according to claims 1-3, **characterized in that** thin active metal films obtained by magnetron or thermal evaporation in air to form a natural or artificially obtained oxide layer selected from Ti/TiO₂ and Zn/ZnO, Zr/ZrO₂, Cr/CrₓO_{y}.

8. The biosensor according to claims 1-3, **characterized in that** the semiconductor surfaces containing an oxide layer selected from Si/SiO₂, Ge/GeO₂ are used for membrane formation.

9. The biosensor of claims 1-8, wherein the biosensor is used to identify a membrane damaging protein agent.

10. The biosensor according to claims 1-8, **characterized in that** its sensitivity to membrane-damaging proteins is altered by altering the composition of phospholipids.

11. The biosensor according to claims 1-8, **characterized in that** its sensitivity to concentration of membrane damaging protein can be increased by using self-assembled monolayer.

12. The biosensor of claims 1-11, wherein the formed sensitive phospholipid layer can be removed and formed one or more times on the same surface.
